# EUROPEAN PATENT APPLICATION

(11) **EP 1 915 988 A1**
(43) Date of publication of application: **30.04.2008**
(21) Application number: 06783012.5
(22) Date of filing: 18.08.2006
(51) Int. Cl.: A61K 9/20

(54) **TABLET HAVING MULTIPLE DRUG-CONTAINING SEGMENTS**

(30) Priority: 18.08.2005 JP 2005237370; 18.08.2005 JP 2005237371
(71) Applicant: Teijin Pharma Limited, Chiyoda-ku, Tokyo 100-0013 (JP)
(72) Inventor: NARASAKI, Masahiko, Teijin Pharma Limited, Iwakuni-shi, Yamaguchi 7400014 (JP); AKUTAGAWA, Tomoya, Teijin Pharma Limited, Iwakuni-shi, Yamaguchi 7400014 (JP); OKU, Reiko, Chiyoda-ku, Tokyo 1000013 (JP)
(74) Representative: Hallybone, Huw George
(86) International application number: PCT/JP2006/316660
(87) International publication number: WO 2007/021033

(57) **Abstract**

A tablet which is either a two-layer tablet comprising a layer A and a layer B or a three-layer tablet comprising a layer A, a layer B and a layer C in that order, wherein the layer B is a medicinal additive layer, the layer A and the layer C are drug layers that are segmented on the layer B, and the sections of the layer A and the layer C are held on the layer B and contain either the same or different drugs. Also, a scored tablet wherein splitting at one of the score lines produces two fragments with equal drug contents and splitting at the other score line produces two fragments with unequal drug contents. In addition, a scored tablet wherein splitting at the score lines exposes cross-sections with different drug release-controlling functions.

## Description

### Technical Field

The present invention relates to a tablet for medical use having multiple drug-containing sections. More specifically, it relates to a tablet which, as a single tablet, can separately and stably retain multiple drugs that must be kept from mutual contact. The invention further relates to a scored tablet that, even after splitting, produces fragments that stably retain a plurality of different drugs that must be kept from mutual contact. The invention still further relates to a scored tablet that retains a plurality of different drugs in a single tablet but that, when split, easily produces fragments containing only one type of drug. The invention yet further relates to a scored tablet that is a single tablet yielding fragments with different drug contents depending on the manner of splitting, and to a scored tablet with varying drug release properties depending on the location of splitting.

### Background Art

Scored tablets are tablets that may be split by a pharmacist or patient according to the dosage, and they are used to optimize dosage management and increase flexibility of prescription (for example, see Japanese Unexamined Patent Publication No. 9-104619 and USP 5520929). Pharmaceutical preparations that can be split into four or more sections have recently been proposed for better optimal dosing and reduced storage space. For example, a 6-scored disc-shaped tablet is disclosed in USP 4503031, and an 8-scored disc-shaped tablet is disclosed in USP 5756124. Also, 4-scored bar-shaped tablets are disclosed in International Patent Publication No. WO 89/05624 and International Patent Publication No. WO 01/00176. However, all of these conventional scored tablets contain equal drug amounts in each split fragment. Moreover, these scored tablets do not have multiple score lines and varying properties depending on the location of splitting.

### DISCLOSURE OF THE INVENTION

It is a first object of the invention to provide a tablet that can stably retain multiple drugs of the same type or different types, such as multiple drugs of different types that must be kept from mutual contact.

It is a second object of the invention to provide a scored tablet that, in a single tablet, separately retains multiple drugs that must be kept from mutual contact, and that, even after splitting, produces fragments that stably retain the multiple drugs that must be kept from mutual contact.

It is a third object of the invention to provide a scored tablet that, in a single tablet, separately retains multiple drugs that may be kept from mutual contact, but that after splitting produces fragments each containing only a single type of drug.

It is yet another object of the invention to provide a coated tablet which is any of the aforementioned tablet types that, after splitting, retains the function of avoiding disadvantage for the patient by contact of the drug with the oral cavity or esophagus.

It is a fourth object of the invention to provide a scored tablet which has two score lines and is designed in such a manner that the combination of drug contents differs in the fragments obtained by splitting at each score line.

It is a fifth object of the invention to provide a scored tablet which has two score lines, wherein the drug release property differs in the fragments obtained by splitting at each score line.

The problems referred to above are solved by the present invention, which will now be explained in detail.

Specifically, the first aspect of the invention is a tablet that is either a two-layer tablet comprising a layer A and a layer B or a three-layer tablet comprising a layer A, a layer B and a layer C, wherein
the layer B is a medicinal additive layer that optionally includes a drug,
the layer A is a drug layer that optionally includes a medicinal additive and is segmented on the layer B,
each of the segmented sections of the layer A is held by being attached to the layer B and contains the same or different drugs,
when a layer C is present, it is also a drug layer that optionally includes a medicinal additive and is segmented on the layer B, and
each of the segmented sections of the layer C is held by being attached to the layer B and contains the same or different drugs. Optionally, the drug in at least one of the regions of a layer A or a layer C may be a drug that must not contact with the drugs in the other regions of the layer A or the layer C.

The second aspect of the invention is a tablet according to the first aspect of the invention having one or more score lines on one or both sides of a layer B,
wherein the splitting locations of a layer A, and of a layer C if it is present, are at the location(s) of the score line(s) on the layer B, or at the corresponding location(s) on the back.

The third aspect of the invention is a tablet that is either a two-layer tablet comprising a layer A and a layer B or a three-layer tablet comprising a layer A, a layer B and a layer C, wherein
the layer B is a medicinal additive layer that optionally includes a drug and has one or more score lines on one or both sides,
the layer A is a drug layer that optionally includes a medicinal additive and is segmented at the location(s) of the score line(s) on the layer B, or at the corresponding location(s) on the back,
each of the segmented sections of the layer A is held by being attached to the layer B and contains the same or different drugs,
when a layer C is present, it is also a drug layer that optionally includes a medicinal additive and is segmented at the location(s) of the score line(s) on the layer B, or at the corresponding location(s) on the back,
each of the segmented sections of the layer C is held by being attached to the layer B and contains the same or different drugs, and
the drug in at least one of the regions of the layer A or the layer C is a drug that is different from the drug(s) in the other region(s) of the same layer, and the drugs of the layer A or the layer C are situated in such a manner that splitting the tablet along at least one of the score lines produces fragments each containing only one type of drug.

The invention also encompasses a tablet according to the aforementioned first to third aspects of the invention wherein the tablet is entirely coated, the coating layer has the function of preventing disadvantage for a patient resulting from contact of the drug with the oral cavity or esophagus, and the function of the coating layer is retained even after splitting.

The fourth aspect of the invention is a tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein splitting at one of the score lines produces two fragments with equal drug contents and splitting at the other score line produces two fragments with unequal drug contents.

The fifth aspect of the invention is a tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein the entirety of the tablet is covered with a coating layer that is insoluble in the gastrointestinal tract or a soluble coating layer that has a drug release-controlling function (for example, a rapid dissolving, rapid disintegrating, enteric-coated, sustained-release or time-release property), and
the connecting section with score line notches is structured so that when the tablet is split at the two score lines, cross-sections with different drug release control functions are exposed.

The tablet of the invention as described above has a simple structure and therefore can achieve the aforementioned objects with low production cost.

### Brief Description of the Drawings

Fig. 1 shows a concrete example of a 3-layer 2-segment tablet according to the invention. (a) is a top view and (2) is a side view.
Fig. 2 shows a concrete example of a 2-layer 2-segment tablet according to the invention. (a) is a top view and (2) is a side view.
Fig. 3 shows a concrete example of a 2-layer 2-segment tablet according to the invention. (a) is a top view and (2) is a side view.
Fig. 4 shows a concrete example of a 3-layer tablet according to the invention, in a side view.
Fig. 5 shows a concrete example of a 2-layer 4-segment tablet according to the invention. (a) is a top view and (2) is a side view.
Fig. 6 shows a concrete example of a 3-layer 4-segment tablet according to the invention. (a) is a top view and (2) is a side view.
Fig. 7 shows a concrete example of a 3-layer 4-segment tablet according to the invention. (a) is a top view and (2) is a side view.
Fig. 8 shows a concrete example of a 2-layer 4-segment tablet according to the invention. (a) is a top view and (2) is a side view.
Fig. 9 shows a concrete example of a 3-layer 4-segment tablet according to the invention. (a) is a top view and (2) is a side view.
Fig. 10 shows side views of four different 2-layer tablets according to the invention. The four tablets differ in the presence or locations of score lines.
Fig. 11 shows a concrete example of a 3-layer 4-segment tablet according to the invention. (a) is a top view and (2) is a side view.
Fig. 12 shows a concrete example of a 3-layer 4-segment tablet according to the invention. (a) is a top view and (2) is a side view.
Fig. 13 shows side views of three different 2-layer tablets according to the invention. The three tablets differ in the locations of the score lines. The peripheral dotted lines represent coating layers.
Fig. 14 shows a concrete example of a 3-layer 2-segment tablet according to the invention. (a) is a top view and (2) is a side view. The peripheral dotted lines represent coating layers.
Fig. 15 shows a concrete example of a 2-layer 2-segment tablet according to the invention. (a) is a top view and (2) is a side view. The peripheral dotted lines represent coating layers.
Fig. 16 shows a concrete example of a 2-layer 2-segment tablet according to the invention. (a) is a top view and (2) is a side view. The peripheral dotted lines represent coating layers.
Fig. 17 shows a side view of a concrete example of a 3-layer tablet according to the invention. The peripheral dotted lines represent coating layers.
Fig. 18 shows a concrete example of a 2-layer 4-segment tablet according to the invention. (a) is a top view and (2) is a side view. The peripheral dotted lines represent coating layers.
Fig. 19 shows a concrete example of a 3-layer 4-segment tablet according to the invention. (a) is a top view and (2) is a side view. The peripheral dotted lines represent coating layers.
Fig. 20 shows a concrete example of a 2-layer 4-segment tablet according to the invention. (a) is a top view and (2) is a side view. The peripheral dotted lines represent coating layers.
Fig. 21 shows a concrete example of the fourth aspect of the invention. (a) is a top view and (2) is a side view.
Fig. 22 shows another concrete example of the fourth aspect of the invention. (a) is a top view and (2) is a side view. The numbered sections are drug-containing sections.
Fig. 23 shows a concrete example of the fifth aspect of the invention. (a) is a top view and (2) is a side view. The peripheral dotted lines represent coating layers.

### Best Mode for Carrying Out the Invention

The first aspect of the invention is a tablet that is either a two-layer tablet comprising a layer A and a layer B or a three-layer tablet comprising a layer A, a layer B and a layer C,
wherein
the layer B is a medicinal additive layer that optionally includes a drug,
the layer A is a drug layer that optionally includes a medicinal additive and is segmented on the layer B (hereinafter referred to as "segmented region"),
each of the segmented sections of the layer A is held by being attached to the layer B and contains the same or different drugs,
when a layer C is present, it is also a drug layer that optionally includes a medicinal additive and is segmented on the layer B (hereinafter referred to as "segmented region"), and
each of the segmented sections of the layer C is held by being attached to the layer B and contains the same or different drugs.

The size of the tablet is not a problem so long as the construction described above can be realized without any inconveniences when administered as a tablet. The shape of the tablet is also not particularly restricted so long as it can be easily produced by an ordinary tabletting machine or a semi-manual tabletting machine, and disk-shaped tablets are typical examples of ordinary "tablets". Triangular or hexagonal columnar or cuboid shapes with low heights may also be mentioned as typical examples, but the shapes are not particularly restricted so long as the basic function of each layer as explained below is not impeded.

The shape of a layer B is basically preferred to be flat on the upper and lower surfaces, but it may be corrugated or irregular if in a form that can hold each of the segmented regions of a layer A or a layer C. Especially with a 2-layer tablet, the opposite side of the layer A is not restricted so long as it can be used as one side of the tablet, since it does not need to anchor any layer.

A layer A or a layer C may be composed only of a drug as the active component or may further contain a medicinal additive, but because it constitutes part of the tablet of the invention it must be a solid form that maintains a consistent shape. It is preferred for the drug to be evenly dispersed even when medicinal additives are included.

The medicinal additives in the layer B or the medicinal additives optionally included in the layer A or the layer C are not particularly restricted, so long as they are pharmaceutically acceptable and do not interfere with stable maintenance of the shape of the tablet. Specifically, there may be used crystalline cellulose, lactose, hydroxypropylcellulose, hydroxypropylmethylcellulose, partially gelatinized starch, starch, erythritol, mannitol, sorbitol, trehalose, light silicic anhydride or mixtures or granules of the above, to mask bitterness or irritation; and lubricants, disintegrators, fluidizing agents and the like may also be included to improve the producibility, moldability and formulating function. In order to maintain or exhibit an enteric coated function, there may be used methacrylic acid copolymer, cellulose acetate phthalate, carboxymethylethylcellulose, hydroxypropylmethylcellulose phthalate, hydroxypropylmethylcellulose acetate succinate, hydroxypropylmethylcellulose and their mixtures, with addition of plasticizers; and in order to maintain or exhibit a sustained-release function, there may be used hydroxyethylcellulose, ethylcellulose, hydroxypropylmethylcellulose phthalate, hydroxypropylmethylcellulose acetate succinate, carboxyvinyl polymer or their mixtures, with addition of plasticizers. Titanium oxide or the like may also be added to the component of the layer B to maintain light stability after splitting.

A layer A or a layer C is segmented on a layer B. The width of the segments may be such as to substantially avoid contact between the adjacent segmented regions. That is, it must be a width so that the drugs that must be kept from mutual contact will not contact each other during storage, when removed from the PTP sheet or when taken in hand for administration. Although the layer A or the layer C is segmented, the segmented regions are held by the layer B by attachment to the layer B, and therefore do not separate.

However, the shapes of the sections other than the surfaces attached to the layer B of the segmented regions of the layer A or the layer C may be freely designed so long as they do not hinder production. They may be cuboid or hemispherical, for example. Also, the shapes of the segmented regions do not need to be the same, and there is also no need to match the shapes of the segmented regions of the layer A and the layer C.

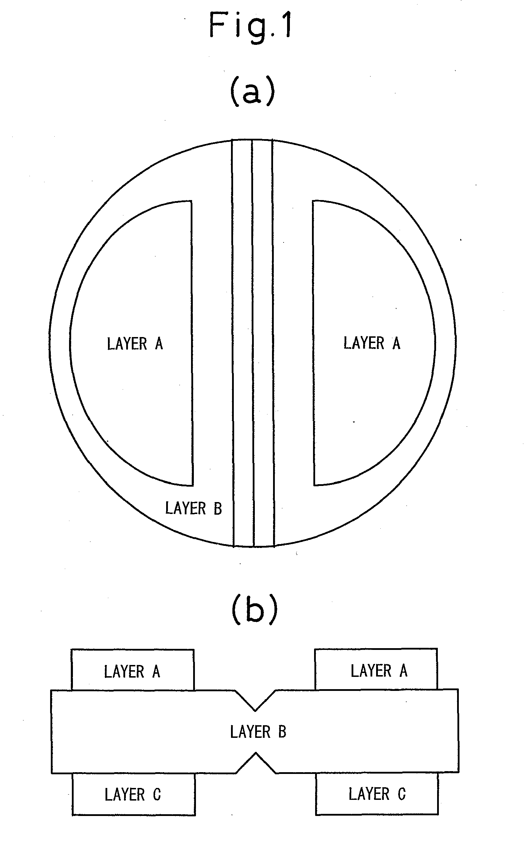

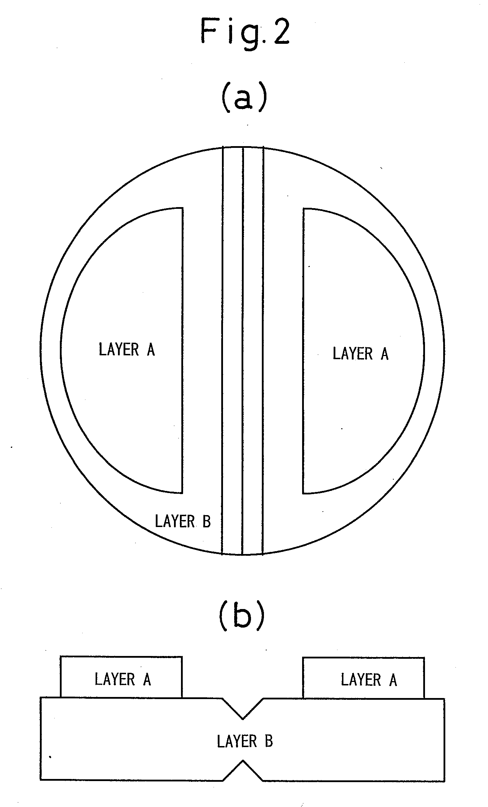

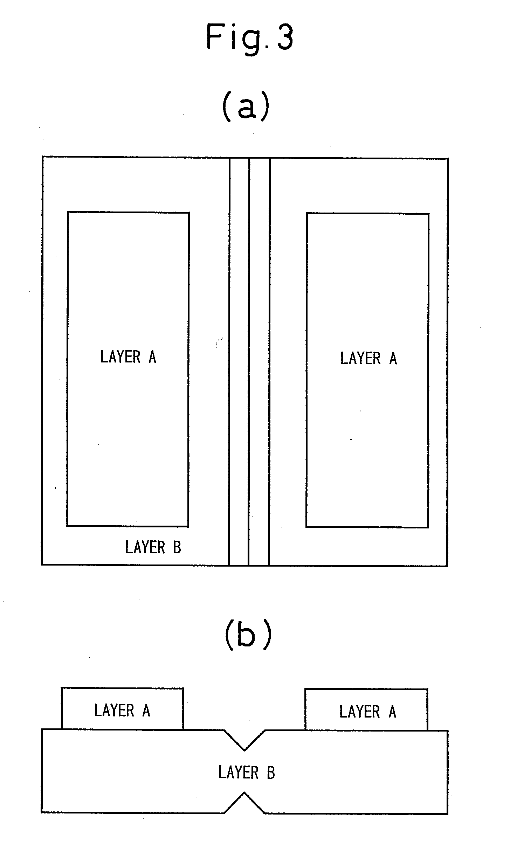

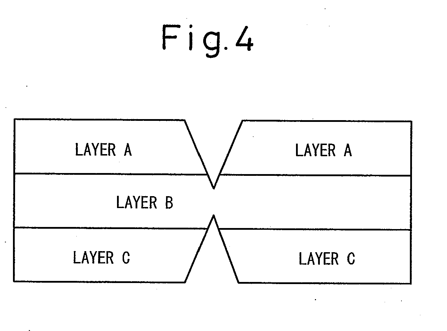

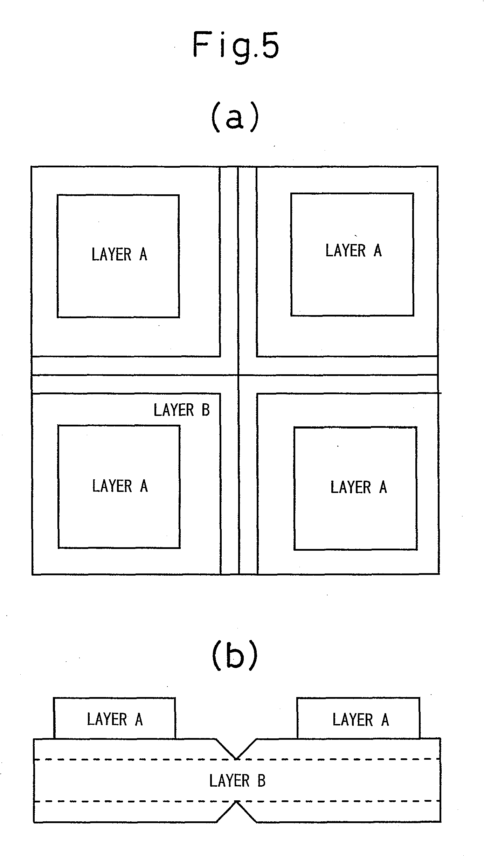

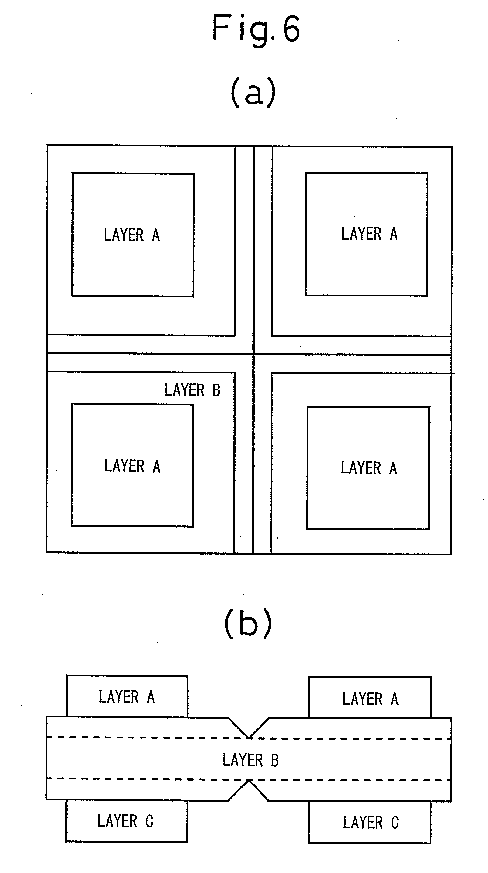

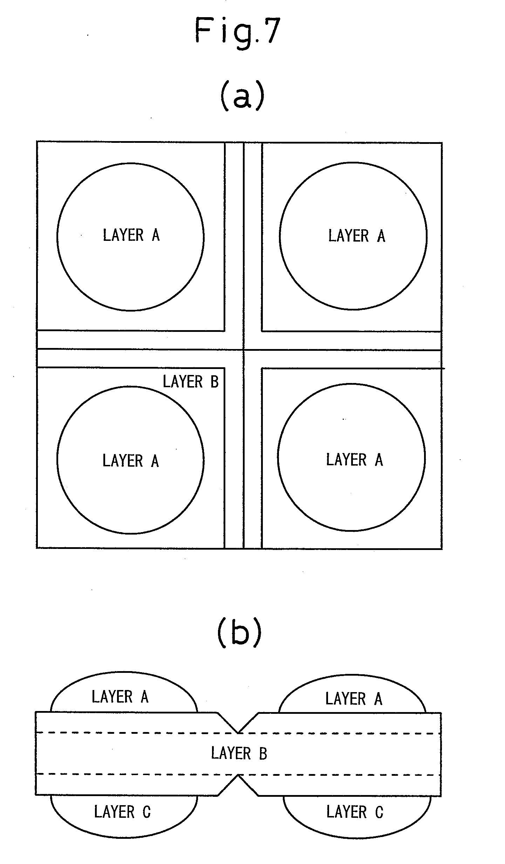

Specific examples of tablets according to the invention will now be explained with reference to the accompanying drawings, with the implicit understanding that they are only for illustration. Fig. 1 shows a 3-layer tablet where a layer B is disc-shaped and the segmented regions of a layer A and a layer C are semicircular. The tablet shown in Fig. 2 is a 2-layer tablet of the same type. Fig. 3 shows a 2-layer tablet where a layer B is square columnar and the segmented regions of a layer A is cuboid. Fig. 4 shows three layers with shapes similar to Fig. 3, but this tablet is characterized in that the shapes of the segmented sections of a layer A and a layer C are integrated with the score lines on a layer B. Fig. 5 shows a 4-segment 2-layer tablet corresponding to the 2-segment 2-layer tablet shown in Fig. 3. The four segmented regions of a layer A have the same square columnar shape. Fig. 6 shows a corresponding 3-layer tablet. Fig. 7 shows the tablet of Fig. 6 wherein the segmented regions of a layer A and a layer C are hemispherical. All of the tablets in Fig. 1 to Fig. 7 are scored tablets, but corresponding non-scored tablets also fall within the scope of the first aspect of the invention.

Different drugs may be used as the drug of a layer A, the drug of a layer B when it contains a drug and the drug of a layer C when it is present. The drugs used as the active components are not particularly restricted so long as they can be formed into a tablet according to the invention by ordinary production processes, and as examples there may be mentioned one or more active components selected from central nervous system drugs, peripheral nervous system drugs, circulatory organ drugs, digestive organ drugs, hormone agents, urogenital drugs, hematological/humoral agents, metabolic disease drugs, gout therapeutic agents, antitumor agents, anti-allergic agents, bronchodilators, antibiotics, antibacterial agents, antiviral drugs, vulnerary substances, antispastic drugs, anti-cholinergic agents, antihistamines, anti-inflammatory drugs, anticholesteremic drugs, antilipidemic drugs, anorectic agents, stimulants, anticoagulants, antacids, chemotherapeutic agents, nutritional supplements, diagnostic agents, narcotic/analeptic agents, analgesics, antitussive agents, expectorants and the like. As specific examples there may be mentioned one or more active components selected from the group consisting of ascorbic acid, acetaminophen, ethenzamide, ambroxol hydrochloride, alendronate, febuxostat, clenbuterol hydrochloride, ethyl icosapentate, tacalcitol, picosulfate, alfacalcidol, the compounds mentioned in International Patent Publication No. WO 99/26918, the compounds mentioned in International Patent Publication No. WO 01/53291, the compounds mentioned in International Patent Publication No. WO 99/25686, and salts and/or hydrates of the above. The drug in a layer A and the drug in a layer B when the layer B contains a drug, or the drug in a layer C when the layer C is present and the drug in a layer B when the layer B contains a drug, are preferably a combination that is not problematic even if they come into mutual contact. Most typically, however, a layer B will contain no drug.

On the other hand, the drug of at least one of the segmented regions of a layer A or a layer C according to the first aspect of the invention may be another drug which must not come into contact with a drug in the other region of the layer A or the layer C.

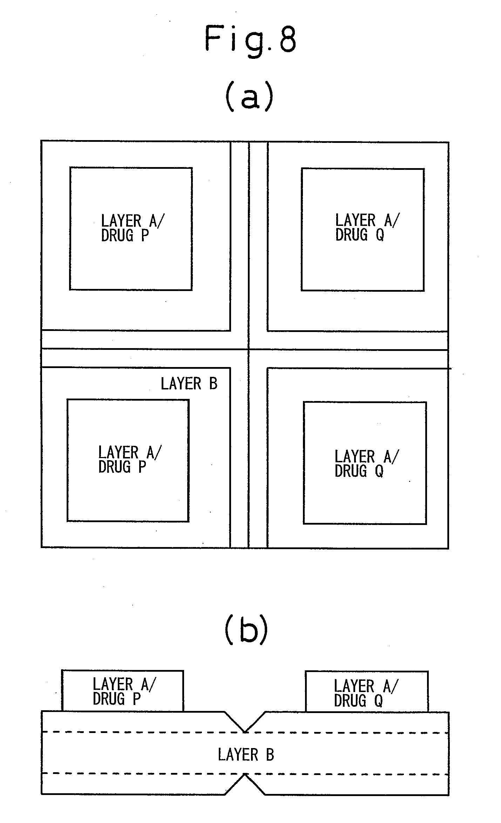

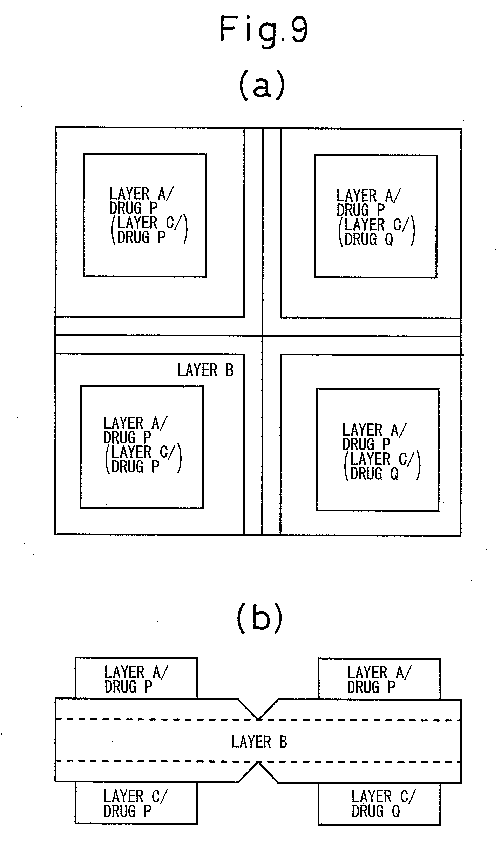

This will be explained referring to the tablet shown in Fig. 8. The drugs that must not come into contact are designated as a drug P and a drug Q. In the tablet shown in Fig. 8, the drug P is located in two of the segmented regions of layer A while the drug Q is located in the other two segmented regions. That is, the drugs are kept in a stable separated state in the tablet. In the right half of the 3-layer tablet shown in Fig. 9, a drug P and a drug Q are located in segmented regions corresponding to layer A and layer C which are separated by layer B, and the same effect is exhibited.

Here, the combination of drugs that must be kept from mutual contact may be, for example, codeine and aspirin or trimetadione and ethotoin. These combinations must be separated to prevent the inconveniences of transesterification and humidification/liquefaction, respectively. Other drugs that are subject to hydrolysis or oxidation undergo alteration when contacted with acidic drugs or basic drugs. Drugs susceptible to hydrolysis include procaine hydrochloride, warfarin, benzylpenicillin, phenobarbital and diazepam, while drugs susceptible to oxidation include epinephrine, ascorbic acid, tocopherol, captopril and sulpyrine.

The construction of the first aspect of the invention produces an effect of allowing two or more types of drugs with poor compatibility to be formulated in the same tablet without coming into mutual contact.

The second aspect of the invention is a scored tablet according to the first aspect of the invention also having one or more score lines on one or both sides of a layer B, wherein the splitting location of a layer A, and of a layer C if it is present, is at the location of the score line on the layer B, or at the corresponding location on the back.

The shape and size of the tablet is still essentially the same as the first aspect, but the shape must be large enough to facilitate splitting of the scored tablet and thin enough not to cause difficulty in splitting. The shape of a layer B and the shapes of the sections of a layer A or a layer C other than the surfaces connecting with the layer B at each segmented region may be the same as for the first aspect, but they must be such as not to constitute a hindrance against splitting only at the score line locations.

According to the second aspect of the invention, one or more score lines are provided on one or both sides of a layer B. The score lines are sometimes referred to as grooves, and grooves are included by the term "score lines" throughout the present specification. Provision of score lines in the tablet and ordinary forms thereof are technical common knowledge in the art. Thus, the fasioning of the score lines according to the invention is not particularly restricted so long as they allow splitting of the tablet without difficulty by a patient or pharmacist, and they can be easily formed based on common technical knowledge to those skilled in the art. Specific examples include the score line forms disclosed in the patent documents mentioned above. When the tablet has multiple score lines, it is necessary for them to be configured so as to allow splitting only at the score line where splitting is intended, and this also presents no special difficulty to a person skilled in the art.

A layer A or a layer C is segmented at the locations of the score lines on a layer B, or at the corresponding sections on the back. The "corresponding sections on the back" means the corresponding sections on the side of a layer B without the score lines, when the score lines of the layer B are formed on only one side.

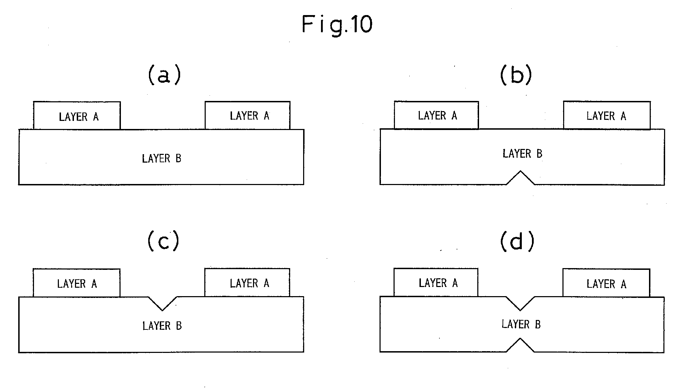

At top left of Fig. 10, there is shown a 2-layer tablet without score lines; at bottom left, there is shown a 2-layer tablet with a layer A segmented at the location of a score line; at top right, there is shown a 2-layer tablet with a layer A segmented at the corresponding section under the location of a score line; and at bottom right, there is shown a 2-layer tablet with score lines on both sides of a layer B and with a layer A segmented at their location.

The segmented regions of a layer A or a layer C do not all need to be in the regions defined by the score lines on a layer B (including regions defined by corresponding locations on the side without score lines, when only on one side has score lines).

The width of the segments according to the first aspect of the invention as described above are not necessarily sufficient for the second aspect of the invention. Splitting of the tablet at a score line locations does not always result in consistent breakage, and some degree of variation is unavoidable. The segmented regions of a layer A or a layer C must therefore be sufficiently separate from the locations of the score lines on a side or the corresponding locations on the back, so as not only to not interfere with the score line function but also to be unaffected by variations in splitting. Therefore, the phrase "segmented at the locations of the score lines on layer B, or at the corresponding locations on the back" implies being segmented by a sufficient width as described above. This provides an effect of allowing accurate splitting without variation in drug doses when the tablet is split.

According to this second aspect of the invention there is provided a scored tablet that, upon splitting, produces fragments containing a subset of the drugs contained in the tablet before splitting.

By preparing a 3-layer scored tablet wherein each of one or more pairs of two types of drugs that must be kept from mutual contact is located in the corresponding segmented regions of a layer A and a layer C that are separated by a layer B, the state in which the two types of drugs that must be kept from mutual contact are located in the corresponding segmented regions of the layer A and the layer C that are separated by the layer B is maintained even after splitting. That is, an effect is exhibited whereby the two types of drugs that must be kept from mutual contact are stably retained even in the split fragments.

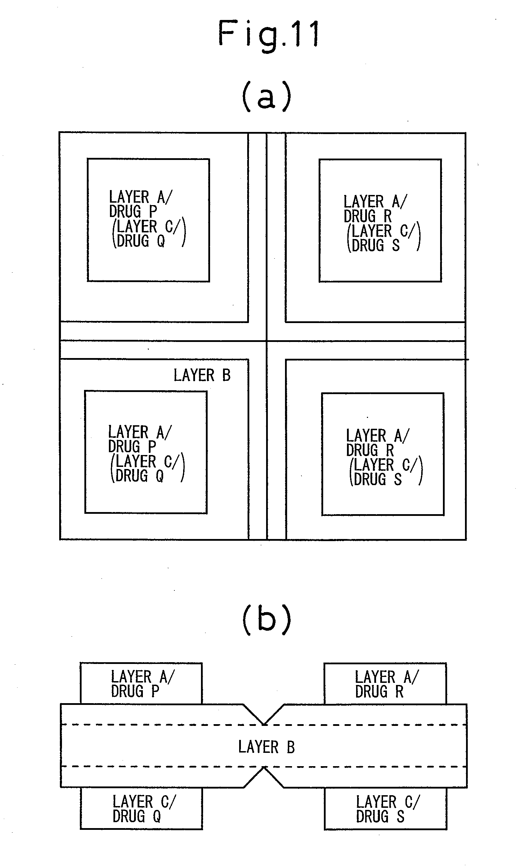

This will be explained referring to the tablet shown in Fig. 11. Here, a drug P and a drug Q must be kept from mutual contact, and a drug R and a drug S must also be kept from mutual contact. In the tablet shown in Fig. 11, each of two pairs of two types of drugs that must be kept from mutual contact is located in corresponding segmented regions of a layer A and a layer C separated by a layer B, and this state is maintained even after splitting. A drug P is present in two segmented regions in the tablet of Fig. 11, but it is still located in the same layer (layer A).

Another embodiment of the second aspect of the invention is a scored tablet having all of the pairs of the two types of drugs that must be kept from mutual contact located in the same layer. In this case, a scored tablet is provided designed with a relationship between the positioning of the drugs in a layer A or a layer C and the score lines such that splitting results in separation of the pairs of the two types of drugs that must be kept from mutual contact into different fragments. This includes 3-layer scored tablets wherein all of the segmented regions of a layer C contain the same drugs as the corresponding drugs of a layer A that is separated by a layer B. Typically, the second aspect of the invention will involve only one pair of the two types of drugs that must be kept from mutual contact, and more typically the scored tablet will contain no drugs other than such a pair of drugs. Such a scored tablet provides increased flexibility of formulation.

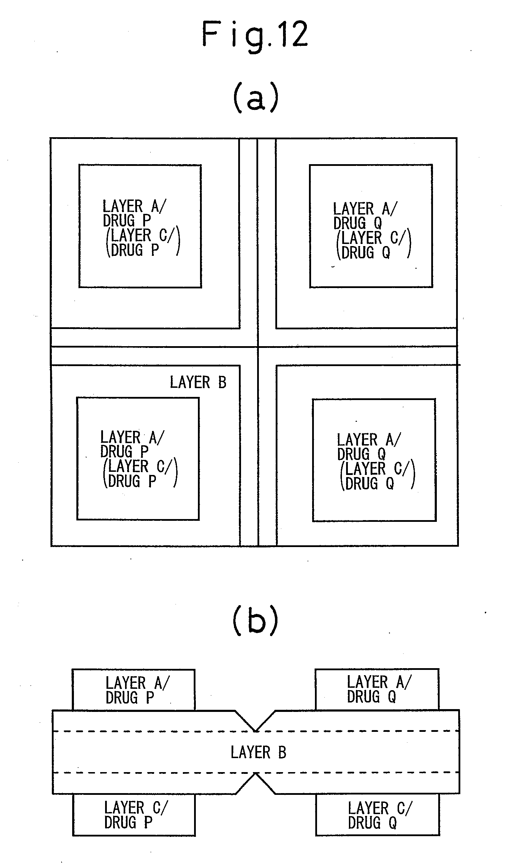

An example of such a tablet is shown in Fig. 12. In this tablet, a drug P and a drug Q are located in the same layer (layer A and layer C). All of the segmented regions of a layer C contain the same drugs as the corresponding drugs in a layer A that is separated by a layer B. This tablet comprises only one pair of two types of drugs that must be kept from mutual contact. Also, the layer A and the layer C contain no drugs other than such a pair of two types of drugs that must be kept from mutual contact. Splitting the tablet along the score line running vertically in the drawing, produces a fragment containing only a drug P and a fragment containing only a drug Q. However, splitting the tablet along the score line running horizontally in the drawing, produces fragments wherein half-doses of a drug P and a drug Q are stably and separately retained.

The third aspect of the invention is a tablet that is either a two-layer tablet comprising a layer A and a layer B or a three-layer tablet comprising a layer A, a layer B and a layer C, wherein
the layer B is a medicinal additive layer that optionally includes a drug and has one or more score lines on one or both sides,
the layer A is a drug layer that optionally includes a medicinal additive and is segmented at the location(s) of the score line(s) on the layer B, or at the corresponding location(s) on the back,
each of the segmented sections of the layer A is held by being attached to the layer B and contains the same or different drugs,
when a layer C is present, it is also a drug layer that optionally includes a medicinal additive and is segmented at the location(s) of the score line(s) on the layer B, or at the corresponding location(s) on the back,
each of the segmented sections of the layer C is held by being attached to the layer B and contains the same or different drugs, and
the drug in at least one of the regions of the layer A or the layer C is a drug that is different from the drug(s) in the other region(s) of the same layer, and the drugs of the layer A or the layer C are situated in such a manner that splitting the tablet along at least one of the score lines produces fragments each containing only one type of drug.

The size and shape of the tablet and the definitions of the terms "layer A", "layer B", "layer C", "medicinal additive", "score line" and "segmented at the location(s) of the score line(s), or at the corresponding location(s) on the back" according to the third aspect of the invention are the same as for the scored tablet described above. Although these drugs do not have the restriction of "must be kept from mutual contact", a plurality of drugs that must be kept from mutual contact may be contained in different segmented regions, such as other segmented regions on the same layer. This will provide an additional effect whereby two or more types of drugs with poor compatibility may be situated in the same tablet without mutual contact even before splitting.

The situation described above whereby "the drug in at least one of the regions of the layer A or the layer C is a drug that is different from the drug(s) in the other region(s) of the same layer, and the drugs of the layer A or the layer C are situated in such a manner that splitting the tablet along at least one of the score lines produces fragments each containing only one type of drug" can be easily established by a person skilled in the art. Typically, a layer B will contain no drug. This tablet provides increased flexibility of formulation, since it allows a plurality type of drugs to be simultaneously administered before splitting, while produces fragments containing only one type of drug after splitting.

As specific examples there may be mentioned the tablets shown in Fig. 8, Fig. 9 and Fig. 12 above.

The tablets according to the first to third aspects of the invention may comprise a coating layer. A coating layer exhibits the function of avoiding disadvantages for a patient by contact of the drug with the oral cavity or esophagus, for example. Such disadvantages for the patient may include, for example, undesirable bitterness or other taste or odor, side-effects due to unintended drug absorption in the oral cavity, or side-effects due to dissolution of the drug in the oral cavity or esophagus. The coating layer is also provided for the purpose of imparting a drug release-controlling function such as an insoluble, rapid-dissolving, rapid-disintegrating, enteric-coating, sustained-release or time-release property, stabilizing the tablet against moisture and humidity, stabilizing the tablet against biological components such as gastric juice and enzymes with which it comes into contact after administration, or providing light stability.

For masking of undesirable tastes and odors, there may be used hydroxypropylcellulose, hydroxypropylmethylcellulose or the like; for an enteric-coated property there, may be used methacrylic acid copolymer, cellulose acetate phthalate, carboxymethylethylcellulose, hydroxypropylmethylcellulose phthalate, hydroxypropylmethylcellulose acetate succinate, hydroxypropylmethylcellulose or their mixtures, with addition of a plasticizer; for a sustained-release property, there may be used hydroxyethylcellulose, ethylcellulose, hydroxypropylmethylcellulose phthalate, hydroxypropylmethylcellulose acetate succinate, carboxyvinyl polymer or their mixtures, with addition of a plasticizer; and for stabilization against moisture and the like, there may be used ethylcellulose, methacrylic acid copolymers, shellac or polyvinylacetaldiethylamino acetate. For stabilization against biological components such as gastric juice or enzyme, there may be used drug additives with masking, enteric-coating or sustained-release functions, either alone or in combinations; and for light stability, there may be added these coating bases with addition of titanium oxide or the like. A plurality of coating layers of different types may also be provided.

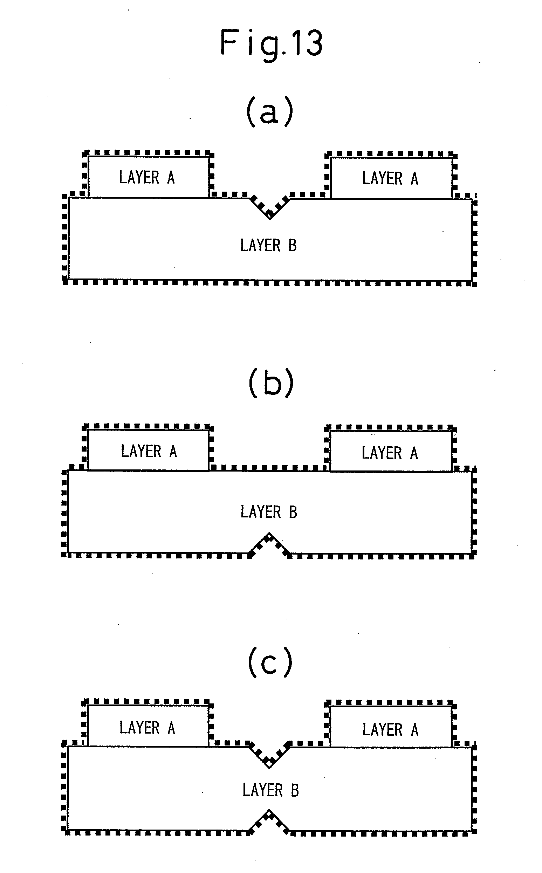

In order to maintain the effect of the coating layer, when the scored tablet has a coating layer, it is preferred for the coating layer to remain on a layer B at the location(s) of the score line(s) or near the corresponding location(s) on the back even after splitting, and for each split section of a layer A or a layer C to be sufficiently separated from the location(s) of the score line(s) or the corresponding location on the back so as to be joined with the layer B or the coating layer without being exposed. An example of such a coated state is shown in Fig. 13. The medicinal additives in a layer B which is exposed after splitting are preferably insoluble or have the same properties as the coating layer, in order to maintain the effect of the coating layer even after splitting.

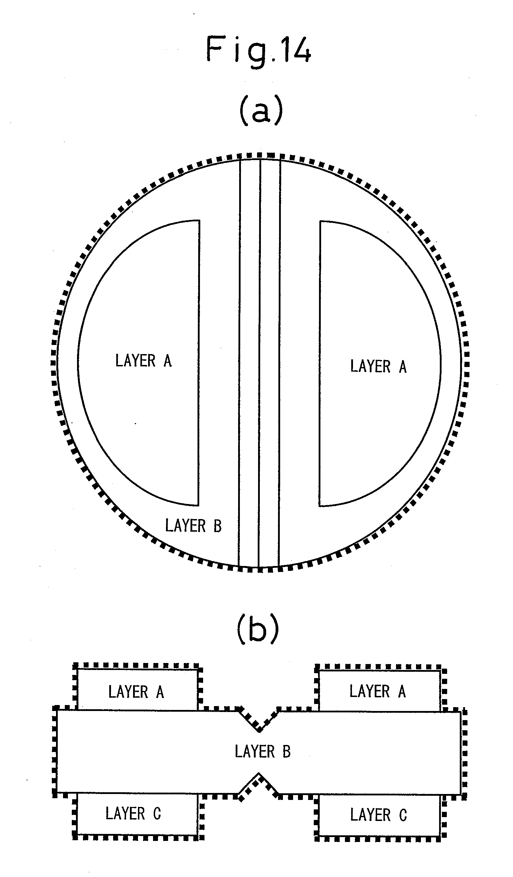

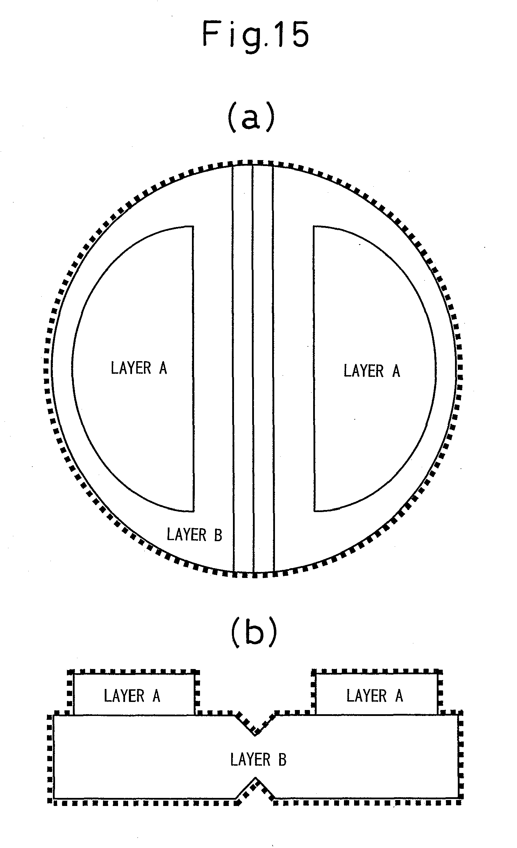

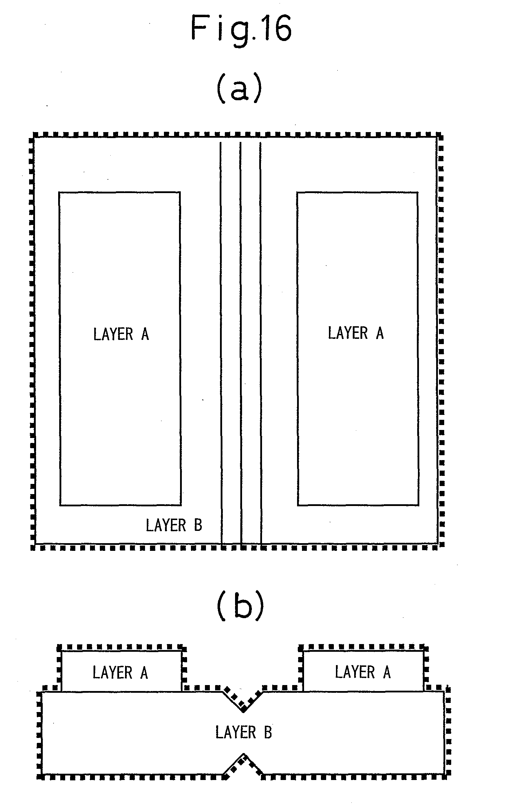

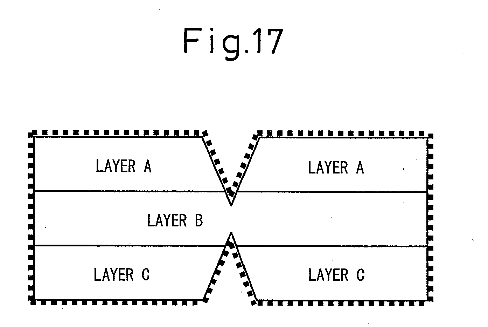

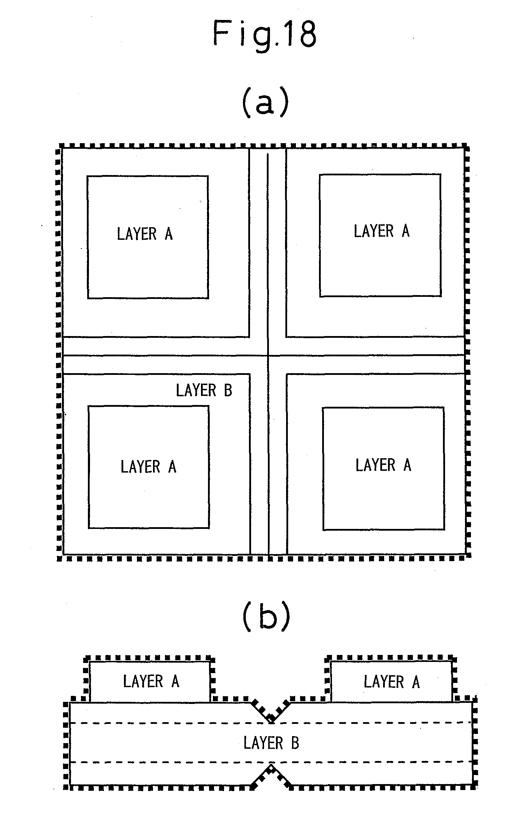

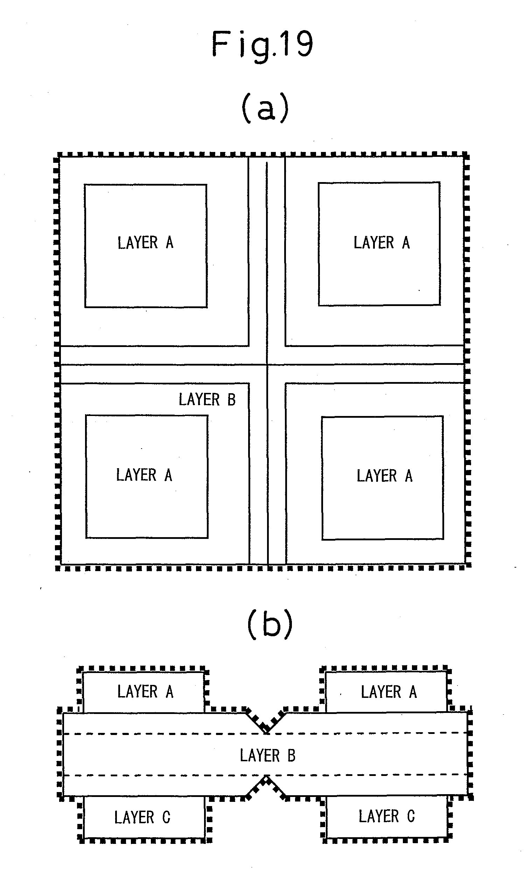

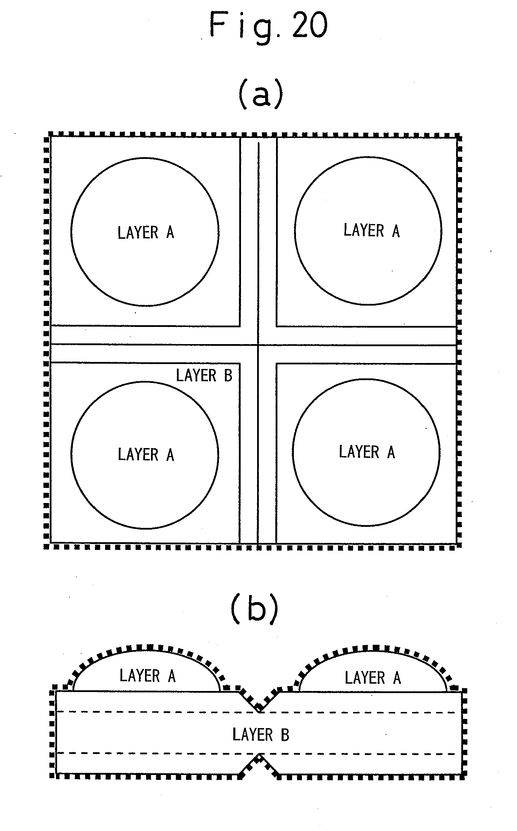

Fig. 14, Fig. 15, Fig. 16, Fig. 17, Fig. 18 and Fig. 19 show coated tablets corresponding to the tablets of Fig. 1, Fig. 2, Fig. 3, Fig. 4, Fig. 5 and Fig. 6, respectively. Fig. 20 shows a 2-layer coated tablet corresponding to the tablet of Fig. 7.

Tablets with a similar effect to that of the invention are known as "dry coated tablets". However, a much higher technical skill is required to produce a dry coated tablet having the same effect as a tablet of the invention. Production of dry coated tablets is associated with high loss and limited speed. It is therefore impossible to avoid the problem of high cost with dry coated tablets.

The fourth aspect of the invention is a tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein splitting at one of the score lines produces two fragments with equal drug contents and splitting at the other score line produces two fragments with unequal drug contents.

The restrictions for the size, thickness and shape of the tablet according to the fourth aspect of the invention are the same as for the tablet according to the first aspect, provided that basic function of each layer described hereunder is not impaired.

It is preferred for the connecting sections to be essentially flat on the upper and lower surfaces, but they may have corrugations or irregularities so long as the drug-containing sections are maintained and so long as splitting can still be accomplished at only one of two score lines.

The drug-containing sections according to the invention may be composed only of drugs as the active components or may further contain medicinal additives, but because they constitute part of the tablet of the invention they must be solid forms with consistent shapes. It is preferred for the drugs to be evenly dispersed even when medicinal additives are included. The shapes of the sections other than the sides of the drug-containing sections adjacent to the connecting sections may be any desired shapes so long as production is not impeded and splitting at the score lines is not hindered. Also, it is not necessary to match the shapes of the different drug-containing sections.

The fourth aspect of the invention has two crossing score lines and one connecting section. Since two score lines are in one connecting section, the shapes of the connecting sections are necessarily those of two crossing strips. They will typically be crossed in perpendicular fashion.

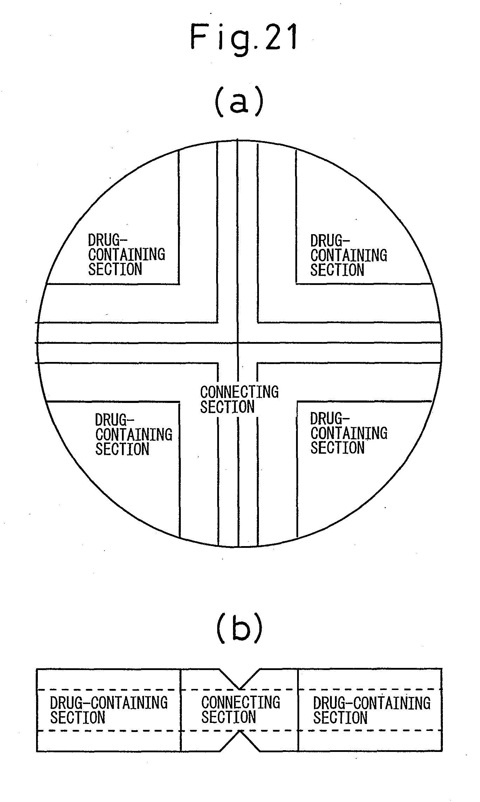

The multiple drug-containing sections preferably have the same shape, and preferably the entire tablet has a form with point symmetry, line symmetry or plane symmetry. Thus, a typical shape for the first aspect of the invention is a quadrant shape viewed from the top, as shown in Fig. 21. Other examples include shapes where the connecting sections are discoid or square cylindrical forms and four drug-containing sections are contiguous with the periphery or four sides of each connecting section.

The restrictions on drugs used as active components for this aspect of the invention are the same as for the first aspect. When the connecting sections include drugs, their combination with the drugs in the drug-containing sections is preferably one that does not result in drawbacks if they come into mutual contact. Typically, however, the connecting section will contain no drug. The medicinal additives in the connecting sections and the medicinal additives that are optionally included in the drug-containing sections are restricted in the same way as the medicinal additives according to the first aspect of the invention.

The fourth aspect of the invention is a tablet having two score lines in the connecting sections, allowing splitting into four segments. The term "score lines" has the same meaning as explained above, and specific examples include the types of score lines disclosed in the aforementioned patent documents. If the connecting sections are flat, for example, the score lines may be on either or both sides.

According to the fourth aspect of the invention, all of the drug-containing sections contain the same drug, a single connecting section is formed, and the drug content in at least one of the drug-containing sections is different from the drug content in the other drug-containing sections. Thus, the drug content is preferably varied by changing the amount of drug per unit volume, without changing the shape of each drug-containing section.

Particularly preferred is a scored tablet wherein splitting at one score line produces two fragments with equal drug contents while splitting at the other score line produces two fragments with unequal drug contents; for example, where the drug contents and positioning of each of the drug-containing sections is such as to yield fragments with two, three or four times a certain drug content.

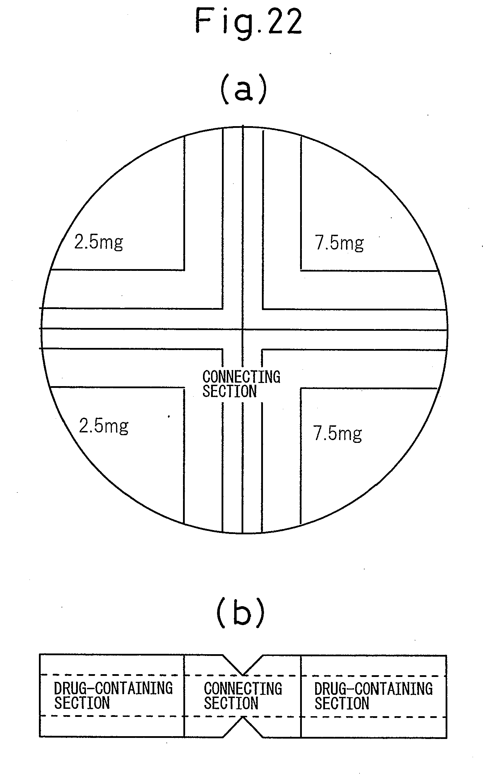

A specific example of the fourth aspect of the invention is the tablet shown in Fig. 22. In this example, a dose of 20 mg is administered if the tablet is not split, whereas splitting along the horizontal score line in the drawing produces two 10 mg fragments. On the other hand, splitting along the vertical score line in the drawing produces a 15 mg fragment and a 5 mg fragment. If the 15 mg fragment is further split in two, two 7.5 mg fragments are produced, whereas if the 5 mg fragment is further split, two 2.5 mg fragments are produced. In other words, various combinations of the fragments allow administration of eight different doses with the same tablet, i.e. 20 mg, 17.5 mg, 15 mg, 12.5 mg, 10 mg, 7.5 mg, 5 mg and 2.5 mg.

The tablet according to the fourth aspect of the invention may also comprise a coating layer. The coating layer is restricted in the same manner as the coating layers of the tablets according to the first to third aspects of the invention. When a coating layer is present, the connecting sections are preferably given a width that will leave a portion of the coating layer on the connecting sections after splitting and avoid exposing the drug-containing sections, so that the effect of the coating layer is maintained even after splitting.

The fifth aspect of the invention is a tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein the entirety of the tablet is covered with a coating layer that is insoluble in the gastrointestinal tract of an organism or a soluble coating layer that has the function of controlling the drug release, and
the connecting section with score line notches is structured so that when the tablet is split at the two score lines, cross-sections with different drug release control functions are exposed.

The function of controlling drug-release at the connecting cross-section may be, for example, an insoluble, rapid-dissolving, rapid-disintegrating, enteric-coating, sustained-release or time-release property. An example of the fifth aspect of the invention is a tablet designed in such a manner that splitting of the tablet along one of the score lines exposes a rapid-dissolving or rapid-disintegrating cross-section while splitting of the tablet at the other score line exposes a sustained-release, enteric-coated or time-release cross-section.

The overall size and shape of the tablet according to the fifth aspect of the invention, as well as the forms of the drug-containing sections, connecting sections and score lines, are the same as for the first aspect of the invention, and the same modes may be mentioned as preferred modes.

Incidentally, the fifth aspect of the invention has the connecting section constructed in such a manner that a rapid-dissolving or rapid-disintegrating cross-section is exposed by splitting the tablet along one score line and a sustained-release, enteric-coated or time-release cross-section is exposed by splitting the tablet along the other score line, but in light of a production standpoint this does not need to be strictly adhered to near the location where the two score lines cross. That is, the region near the location where the two score lines cross may be composed of a sustained-release or enteric-coated material in this example. In other words, a rapid-dissolving, rapid-disintegrating or time-release section may be segmented at the location where the score lines cross. This will still provide the effect of the fifth aspect of the invention, whereby selection of the segmenting score line can result in different drug release properties of the obtained fragments.

For a rapid-dissolving property of the connecting sections there may be used a mixture or granules containing one or more of, for example, lactose, crystalline cellulose, partially gelatinized starch, starch, mannitol, xylitol, erythritol, sorbitol, croscarmellose sodium, carmellose calcium, crospovidone or the like, and for a disintegrating property, the content of disintegrators such as starches, croscarmellose sodium, carmellose calcium, crospovidone, partially gelatinized starch or other starch or readily-soluble polysaccharides such as mannitol, erythritol and the like among the aforementioned additives may be increased.

The amounts of drugs in the drug-containing sections according to the fifth aspect of the invention may be different, but for most purposes they will be the same.

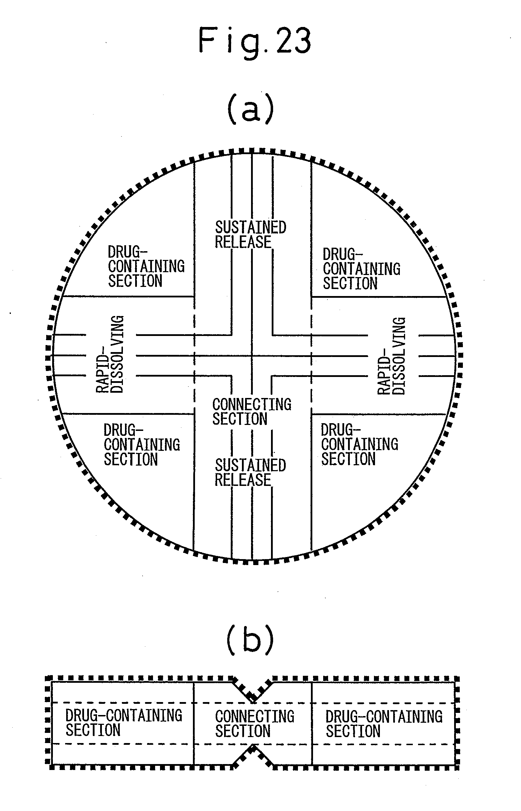

The tablet of Fig. 23 as an example of the fifth aspect of the invention has the same shape as the tablet of Fig. 21, but it is not limited to this shape so long as the function of the fifth aspect of the invention is exhibited. For example, the arc sections at the outer periphery of the drug-containing sections in the tablet of Fig. 23 may instead be straight lines.

### Examples

The present invention will now be described in greater detail by examples, with the understanding that the invention is not limited by these examples.

### Example 1: (Multilayer tabletting method) Upper layer filling → Lower layer filling → Compression molding

An elliptical punching die (F-J0392-1 by Fuji Yakuhin Kikai, KK., with score line on the upper punch) was fitted in a tabletting machine (HT-AP6SS-U by Hata Iron Works Co., Ltd.). The die was packed with 80 mg of a mixture of Dilactose R (product of Freund) with 0.5% magnesium stearate, and 30 mg of Dilactose R (product of Freund) with HPC-L and Blue #1-colored powder was packed onto the top layer. The mixture was compression molded with a precompression punch tip spacing of 1.56 mm and a main compression punch tip spacing of from 1.35 to 1.40 mm, to obtain a tablet having a main drug layer segmented by the score line section of a special score line punch.

### Example 2: (Multilayer tabletting method) Upper layer packing → Lower layer packing → Compression molding

An elliptical punching die (F-J0392-1 by Fuji Yakuhin Kikai, KK., with score line on the upper punch) was fitted in a tabletting machine (HT-AP6SS-U by Hata Iron Works Co., Ltd.). The die was packed with 80 mg of a mixture of Dilactose R (product of Freund) with 0.5% magnesium stearate, and 30 mg of a mixture of Dilactose R (product of Freund) with sodium ascorbate and magnesium stearate was packed on the top layer. The mixture was compression molded with a precompression punch tip spacing of 1.56 mm and a main compression punch tip spacing of from 1.35 to 1.40 mm, to obtain a tablet having a main drug layer segmented by the score line section of a special score line punch.

### Example 3

Granules made of sodium ascorbate, lactose, partially gelatinized starch and hydroxypropylcellulose and granules made of lactose, partially gelatinized starch and hydroxypropylcellulose were mixed with croscarmellose sodium and magnesium stearate, respectively, and used as tabletting powder.

A special oval tablet punching die (upper punch: F-J0375, lower punch: F-J0374, by Fuji Yakuhin Kikai, KK.) was mounted in a tabletting machine (HT-AP6SS-U by Hata Iron Works Co., Ltd.). The die was packed with 120 mg of a tabletting powder containing no febuxostat on the lower layer and 30 mg of a tabletting powder containing febuxostat on the upper layer, and the powder was compression molded to obtain a tablet having a main drug layer segmented by the score line section of a special score line punch.

### Example 4

Granules made of febuxostat, lactose, partially gelatinized starch and hydroxypropylcellulose and granules made of lactose, partially gelatinized starch and hydroxypropylcellulose were mixed with croscarmellose sodium and magnesium stearate, respectively, and used as tabletting powder.

A special oval tablet punching die (upper punch: F-J0375, lower punch: F-J0374, by Fuji Yakuhin Kikai, KK.) was mounted in a tabletting machine (HT-AP6SS-U by Hata Iron Works Co., Ltd.). The die was packed with 120 mg of a tabletting powder containing no febuxostat on the lower layer and 30 mg of a tabletting powder containing febuxostat on the upper layer, and the powder was compression molded to obtain a tablet having a main drug layer segmented by the score line section of a special score line punch.

### Example 5: (Multilayer tabletting method) Upper layer packing → Lower layer packing → Compression molding → Coating

An elliptical punching die (F-J0392-1 by Fuji Yakuhin Kikai, KK., with score line on the upper punch) was fitted in a tabletting machine (HT-AP6SS-U by Hata Iron Works Co., Ltd.). The die was packed with 80 mg of a mixture of Dilactose R (product of Freund) with 0.5% magnesium stearate, and 30 mg of Dilactose R (product of Freund) with HPC-L and Blue #1-colored powder was packed onto the top layer. The mixture was compression molded with a precompression punch tip spacing of 1.56 mm and a main compression punch tip spacing of from 1.35 to 1.40 mm, to obtain a tablet having an upper layer segmented by the score line section of a special score line punch.

The tablet was film-coated using a coating machine (High-Coater Mini, product of Freund). The operating conditions were as follows: Atomizing was carried out with an intake temperature of 68-74°C, an outlet temperature of 44-50°C, a pan rotation of 20-30 rpm, atomizing air at 60 NL/min, pattern air at 30 NL/min and 233 g of coating solution (6% HPMC TC-5 0.12% PEG 6000P aqueous solution), and followed by drying.

### Example 6: (Multilayer tabletting method) Upper layer packing → Lower layer packing → Compression molding Coating

An elliptical punching die (F-J0392-1 by Fuji Yakuhin Kikai, KK., with score line on the upper punch) was fitted in a tabletting machine (HT-AP6SS-U by Hata Iron Works Co., Ltd.). The die was packed with 80 mg of a mixture of Dilactose R (product of Freund) with 0.5% magnesium stearate, and 30 mg of a mixture of Dilactose R (product of Freund) with sodium ascorbate and magnesium stearate was packed onto the top layer. The mixture was compression molded with a precompression punch tip spacing of 1.56 mm and a main compression punch tip spacing of from 1.35 to 1.40 mm, to obtain a tablet having an upper layer segmented by the score line section of a special score line punch.

The tablet was film-coated using a coating machine (High-Coater Mini, product of Freund). The operating conditions were as follows: Atomizing was carried out with an intake temperature of 68-74°C, an outlet temperature of 44-50°C, a pan rotation of 20-30 rpm, atomizing air at 60 NL/min, pattern air at 30 NL/min and 233 g of coating solution (6% HPMC TC-5 0.12% PEG 6000P aqueous solution), and followed by drying.

### Example 7: (Small tablet packing method 1) Lower layer packing → Small tablet packing → Compression molding

A 10 mm flat punching die (with upper and lower score lines) was fitted in a tabletting machine (HT-AP6SS-U by Hata Iron Works Co., Ltd.). The die was packed with 100 mg of a mixture of Dilactose R (Freund) with 0.5% magnesium stearate for use as the lower layer. Two R tablets with a diameter of 3 mm and a weight of 25 mg, containing 2.5 mg and 5.0 mg of febuxostat, were packed onto the lower layer so as to sandwich the score lines, and subjected to compression molding.

### Example 8: (Small tablet packing method 1) Lower layer packing → Small tablet packing → Compression molding

A 10 mm flat punching die (with upper and lower score lines) was fitted in a tabletting machine (HT-AP6SS-U by Hata Iron Works Co., Ltd.). The die was packed with 100 mg of a mixture of Dilactose R (Freund) with 0.5% magnesium stearate for use as the lower layer. Two R tablets with a diameter of 3 mm and a weight of 25 mg, containing 5.0 mg and 10.0 mg of ascorbic acid, were packed onto the lower layer so as to sandwich the score lines, and subjected to compression molding.

### Example 9: (Small tablet packing method 1) Lower layer packing → Small tablet packing → Compression molding

A 10 mm flat punching die (with upper and lower score lines) was fitted in a tabletting machine (HT-AP6SS-U by Hata Iron Works Co., Ltd.). The die was packed with 100 mg of a mixture of Dilactose R (Freund) with 0.5% magnesium stearate for use as the lower layer. Two R tablets with a diameter of 3 mm and a weight of 25 mg, containing 5.0 mg and 10.0 mg of 3-(amidinophenyl)-5-[({[1-(iminoethyl)(4-piperidyl)]methyl}amino)methyl]benzoic acid dihydrochloride trihydrate, were packed onto the lower layer so as to sandwich the score lines, and subjected to compression molding.

### Industrial Applicability

The present invention can be used for manufacturing a pharmaceutical agent.

## Claims

1. A tablet that is either a two-layer tablet comprising a layer A and a layer B or a three-layer tablet comprising a layer A, a layer B and a layer C, wherein
the layer B is a medicinal additive layer that optionally includes a drug,
the layer A is a drug layer that optionally includes a medicinal additive and is segmented on the layer B,
each of the segmented sections of the layer A is held by being attached to the layer B and contains the same or different drugs,
when a layer C is present, it is also a drug layer that optionally includes a medicinal additive and is segmented on the layer B, and
each of the segmented sections of the layer C is held by being attached to the layer B and contains the same or different drugs.

2. The tablet according to claim 1, wherein the drug in at least one of the regions of a layer A or a layer C is a drug that must not contact with the drugs in the other regions of the layer A or the layer C.

3. The tablet according to claim 1 or 2 having one or more score lines on one or both sides of a layer B,
wherein the splitting locations of a layer A, and of a layer C if it is present, are at the location(s) of the score line(s) on the layer B, or at the corresponding location(s) on the back.

4. The tablet according to claim 3, which is a three-layer tablet wherein each of one or more pairs of two types of drugs that must be kept from mutual contact is located in corresponding segmented regions of a layer A and a layer C separated by a layer B.

5. The tablet according to claim 4, wherein one of the two types of drugs that must be kept from mutual contact is located entirely in the same layer.

6. The tablet according to claim 3, wherein each of the pairs of two types of drugs that must be kept from mutual contact is located entirely in the same layer.

7. The tablet according to claim 6, wherein all of the segmented regions of a layer C contain the same drugs as the corresponding drugs of a layer A that is separated by a layer B.

8. The tablet according to any one of claims 2 to 7, wherein the tablet comprises one pair of two drugs that must be kept from mutual contact.

9. The tablet according to claim 8, wherein the layer A and the layer C contain no drugs other than the drugs that must be kept from mutual contact.

10. A tablet that is either a two-layer tablet comprising a layer A and a layer B or a three-layer tablet comprising a layer A, a layer B and a layer C, wherein
the layer B is a medicinal additive layer that optionally includes a drug and has one or more score lines on one or both sides,
the layer A is a drug layer that optionally includes a medicinal additive and is segmented at the location(s) of the score line(s) on the layer B, or at the corresponding location(s) on the back,
each of the segmented sections of the layer A is held by being attached to the layer B and contains the same or different drugs,
when a layer C is present, it is also a drug layer that optionally includes a medicinal additive and is segmented at the location(s) of the score line(s) on the layer B, or at the corresponding location(s) on the back,
each of the segmented sections of the layer C is held by being attached to the layer B and contains the same or different drugs, and
the drug in at least one of the regions of the layer A or the layer C is a drug that is different from the drug(s) in the other region(s) of the same layer, and the drugs of the layer A or the layer C are situated in such a manner that splitting the tablet along at least one of the score lines produces fragments each containing only one type of drug.

11. The tablet according to claim 10, wherein the drug in at least one of the regions of a layer A or a layer C is a drug that must not contact with the drug in another region of the same layer.

12. The tablet according to claim 10 or 11, wherein fragments containing only one type of drug are produced by a single splitting manipulation.

13. The tablet according to any one of claims 1 to 12, wherein the layer B contains no drug.

14. The tablet according to any one of claims 1 to 13, wherein the entirety is covered with one or more coating layers.

15. The tablet according to claim 14, wherein the coating layer has the function of avoiding disadvantage for a patient by contact of the drug with the oral cavity or esophagus.

16. The tablet according to claim 15, wherein the disadvantage for a patient due to contact of the drug with the oral cavity or esophagus is bitterness, irritation, a side-effect due to unintended drug absorption in the oral cavity, or a side-effect due to dissolution of the drug in the oral cavity or esophagus.

17. A tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein splitting at one of the score lines produces two fragments with equal drug contents and splitting at the other score line produces two fragments with unequal drug contents.

18. The tablet according to claim 17, wherein the splitting at one of the score lines produces two fragments with equal drug contents and the splitting at the other score line produces two fragments with drug contents differing by a multiple of two, three or four.

19. The tablet according to claim 17 or 18, wherein the entirety is covered with a coating layer.

20. The tablet according to claim 19, wherein the coating layer has the function of avoiding disadvantage for a patient by contact of the drug with the oral cavity or esophagus.

21. The tablet according to claim 19, wherein the coating layer has a drug release-controlling function.

22. The tablet according to claim 21, wherein the drug release-controlling function is a rapid-dissolving, rapid-disintegrating, enteric-coating, sustained-release or time-release property.

23. A tablet comprising:
a plurality of drug-containing sections that each optionally includes a medicinal additive, and
a single connecting section composed of a pharmaceutically acceptable component, which is contiguous therewith,
all of the drug-containing sections containing the same type of drug, and
the connecting section having two crossing score lines allowing the entire tablet to be split into four sections,
wherein the entirety of the tablet is covered with a coating layer that is insoluble in the gastrointestinal tract or a soluble coating layer that has the function of controlling the drug release, and
the connecting section has the score lines structured so that when the tablet is split at the two score lines, cross-sections with different drug release-controlling functions are exposed.
